# EUROPEAN PATENT SPECIFICATION

(11) **EP 4 168 008 B1**
(45) Date of publication and mention of the grant of the patent: **18.03.2026**
(21) Application number: 21828761.3
(22) Date of filing: 21.06.2021
(51) Int. Cl.: A61K 31/47, A61K 31/277, A61P 35/04

(54) **A COMBINATION OF BELZUTIFAN AND LENVATINIB FOR USE IN TREATING CANCER**
EINER KOMBINATION AUS BELZUTIFAN UND LENVATINIB ZUR VERWENDUNG BEI DER BEHANDLUNG VON KREBS
UNE ASSOCIATION DU BELZUTIFAN ET DU LENVATINIB POUR SON UTILISATION DANS LE TRAITEMENT DU CANCER

(30) Priority: 22.06.2020 US 202063042307 P; 08.02.2021 US 202163146915 P
(43) Date of publication of application: 26.04.2023
(73) Proprietor: Merck Sharp & Dohme LLC, Rahway, New Jersey 07065 (US); Eisai R&D Management Co., Ltd., Tokyo 112-8088 (JP)
(72) Inventor: PERINI, Rodolfo Fleury, North Wales, Pennsylvania 19454-2505 (US); PINHEIRO, Elaine, M., Boston, Massachusetts 02115-5727 (US); WILLEMANN ROGERIO, Jaqueline, Rahway, New Jersey 07065-0907 (US)
(74) Representative: Kilburn & Strode LLP
(86) International application number: PCT/US2021/038172
(87) International publication number: WO 2021/262563

(56) References cited:
- WO-A1-2018/031680
- WO-A1-2020/081695
- US-A1- 2018 140 569
- ERIC JONASCH ET AL: "Belzutifan (MK-6482) for Von Hippel-Lindau Disease? Associated Renal Cell Carcinoma", ASCO 2020 (AMERICAN SOCIETY OF CLINICAL ONCOLOGY), VIRTUAL SCIENTIFIC PROGRAM; MAY 29-31, 2020, AMERICAN SOCIETY OF CLINICAL ONCOLOGY, US, 29 May 2020 (2020-05-29), pages 1 - 28, XP009535918
- JONASCH1 E ET AL: "911PD - A First-in-Human Phase 1/2 Trial of the Oral HIF-2a Inhibitor PT2977 in Patients with Advanced RCC", vol. 30, no. 3170 Suppl. 5, 29 September 2019 (2019-09-29), pages v361 - v362, XP009555040, ISSN: 0923-7534, Retrieved from the Internet <URL:https://www.sciencedirect.com/science/article/pii/S092375341959127X>
- ANONYMOUS CLINICALTRIALS: "A Phase 1, Multiple-Dose, Dose-Escalation and Expansion Trial of PT2977, a HIF-2[alpha] Inhibitor, in Patients With Advanced Solid Tumors Record History | ver. 14: 2020-05-06 | NCT02974738 | ClinicalTrials.gov", 6 May 2020 (2020-05-06), pages 1 - 15, XP093176173, Retrieved from the Internet <URL:https://clinicaltrials.gov/study/NCT02974738?tab=history&a=14#version-content-panel>
- ANON: "Lenvatinib in combination with Everolimus", 16 May 2016 (2016-05-16), pages 1 - 3, XP093176330, Retrieved from the Internet <URL:https://www.fda.gov/drugs/resources-information-approved-drugs/lenvatinib-combination-everolimus>
- ANON: "Kidney Cancer Journal", KIDNEY CANCER JOURNAL, 1 January 2020 (2020-01-01), pages 1 - 32, XP055771484, Retrieved from the Internet <URL:https://www.kidney-cancer-journal.com/assets/kcj_v18n1_final_lo-res_2.5.pdf#page=19> [retrieved on 20210202]
- XU RUI, WANG KESHI, RIZZI JAMES P., HUANG HELI, GRINA JONAS A., SCHLACHTER STEPHEN T., WANG BIN, WEHN PAUL M., YANG HANBIAO, DIXON: "3-[(1 S ,2 S ,3 R )-2,3-Difluoro-1-hydroxy-7-methylsulfonylindan-4-yl]oxy-5-fluorobenzonitrile (PT2977), a Hypoxia-Inducible Factor 2α (HIF-2α) Inhibitor for the Treatment of Clear Cell Renal Cell Carcinoma", JOURNAL OF MEDICINAL CHEMISTRY, AMERICAN CHEMICAL SOCIETY, US, vol. 62, no. 15, 8 August 2019 (2019-08-08), US , pages 6876 - 6893, XP055896754, ISSN: 0022-2623, DOI: 10.1021/acs.jmedchem.9b00719

## Description

### FIELD OF INVENTION

Provided herein are methods for treating renal cell carcinoma (RCC) using a combination of (a) belzutifan and (b) lenvatinib, or a pharmaceutically acceptable salt thereof.

### BACKGROUND OF THE INVENTION

Intratumoral hypoxia is a driving force in cancer progression and is closely linked to poor patient prognosis and resistance to chemotherapy and radiation treatment. Hypoxia-Inducible Factors (HIF-1α and HIF-2α) are transcription factors that play central roles in the hypoxic response pathway. Under normoxic conditions, the tumor suppressor von Hippel-Lindau (VHL) protein binds to specific hydroxylated proline residues and recruits the E3 ubiquition-ligase complex that targets HIF-α proteins for proteasomal degradation. Under hypoxic conditions, HIF-α proteins accumulate and enter the nucleus to stimulate the expression of genes that regulate anaerobic metabolism, angiogenesis, cell proliferation, cell survival, extracellular matrix remodeling, pH homeostasis, amino acid and nucleotide metabolism, and genomic instability. VHL deficiency can also result in accumulated HIF expression under oxygenated conditions (pseudohypoxic conditions). Accordingly, directly targeting HIF-α proteins offers an exciting opportunity to attack tumors on multiple fronts (Keith, et al., Nature Rev. Cancer 12: 9-22, 2012).

Specifically, HIF-2α is a key oncogenic driver in clear cell renal cell carcinoma (ccRCC) (Kondo, K., et al., Cancer Cell, 1:237-246 (2002); Maranchie, J. et al, Cancer Cell, 1:247-255 (2002); Kondo, K., et al., PLoS Biol., 1:439-444 (2003)). In mouse ccRCC tumor models, knockdown of HIF-2α expression in pVHL (von Hippel-Lindau protein) defective cell lines blocked tumor growth comparable to reintroduction of pVHL. In addition, expression of a stabilized variant of HIF-2α was able to overcome the tumor suppressive role of pVHL. Belzutifan, a novel HIF-2α inhibitor with excellent in vitro potency, pharmacokinetic profile and in vivo efficacy in mouse models, has shown encouraging outcomes in patients with advanced renal cell carcinoma (Xu, Rui, et al., J. Med. Chem. 62:6876-6893 (2019).

Von-Hippel Lindau disease (VHL disease) is an autosomal dominant syndrome that not only predisposes patients to kidney cancer (~70% lifetime risk), but also to hemangioblastomas, pheochromocytoma and pancreatic neuroendocrine tumors. VHL disease results in tumors with constitutively active HIF-α proteins with the majority of these dependent on HIF-2 α activity (Maher, et al. Eur. J. Hum. Genet. 19: 617-623, 2011). HIF-2 α has been linked to cancers of the retina, adrenal gland and pancreas through both VHL disease and activating mutations. Recently, gain-of-function HIF-2 α mutations have been identified in erythrocytosis and paraganglioma with polycythemia (Zhuang, et al. NEJM 367: 922-930, 2012; Percy, et al. NEJM 358: 162-168, 2008; and Percy, et al. Am. J. Hematol. 87: 439-442, 2012). Notably, a number of known HIF-2α-target gene products (e.g., VEGF, PDGF, and cyclin D1) have been shown to play pivotal roles in cancers derived from kidney, liver, colon, lung, and brain. In fact, therapies targeted against one of the key HIF-2α regulated gene products, VEGF, have been approved for the treatment of these cancers.

Tyrosine kinases are involved in the modulation of growth factor signaling and thus are important targets for cancer therapies. Lenvatinib is a multiple RTK (multi-RTK) inhibitor that selectively inhibits the kinase activities of vascular endothelial growth factor (VEGF) receptors (VEGFR1 (FLT1), VEGFR2 (KDR) and VEGFR3 (FLT4)), and fibroblast growth factor (FGF) receptors FGFR1, 2, 3 and 4 in addition to other proangiogenic and oncogenic pathway-related RTKs (including the platelet-derived growth factor (PDGF) receptor PDGFRα; KIT; and the RET proto-oncogene (RET)) involved in tumor proliferation. In particular, lenvatinib possesses a new binding mode (Type V) to VEGFR2, as confirmed through X-ray crystal structural analysis, and exhibits rapid and potent inhibition of kinase activity, according to kinetic analysis.

Hypoxic responses lead to the upregulation of genes that promote angiogenesis and vascularization to increase oxygen delivery. This includes the transcriptional activation of vascular endothelial growth factor (VEGF) which can regulate several endothelial cell responses such as activation and proliferation. VEGF tyrosine kinases can block VEGF receptor signaling systemically while HIF-2α inhibition can block tumor VEGF A expression and secretion. The combination of a VEGF TKI and HIF-2α inhibitor enables orthogonal inhibition of VEGFA secretion and signaling without overlapping toxicities. In addition, HIF-2α inhibition can suppress VEGF TKI induced HIF-2α activity and anti-angiogenesis resistance. See, D. Shweiki, A. Itin, D. Soffer, E. Keshet. Vascular endothelial growth factor induced by hypoxia may mediate hypoxia-initiated angiogenesis. Nature, 359 (1992), pp. 843-845; J.A. Forsythe, B.H. Jiang, N.V. Iyer, F. Agani, S.W. Leung, R.D. Koos, G.L. Semenza. Activation of vascular endothelial growth factor gene transcription by hypoxia-inducible factor 1. Mol Cell Biol, 16 (1996), pp. 4604-4613; Hypoxic stress: obstacles and opportunities for innovative immunotherapy of cancer. Chouaib S, Noman MZ, Kosmatopoulos K, Curran MA. Oncogene. 2017 Jan 26;36(4):439-445. doi: 10.1038/onc.2016.225. Epub 2016 Jun 27.

There have been recent advances in the treatment of first line (1L) advanced RCC combining immunomodulators and/or VEGF-TKI(s), and multiple agents also now available for the treatment of patients with second line (2L) RCC. However existing data shows that few patients experience complete response with these agents and nearly all progress. Although these significant advances have led to a change in the treatment paradigm of these patients, there remains an unmet need to improve outcomes for both 1L and 2L+ advanced RCC populations. Currently there is no standard of care (SOC) for post PD-(L)1 inhibitors /post VEGF-TKI 2L+ advanced RCC, and there are no large randomized clinical trials in this setting.

There are several drugs currently available for 2L+ therapy in patients with advanced RCC including nivolumab, cabozantinib, and lenvatinib in combination with everolimus. These agents have shown benefit in terms of outcome compared with single agent everolimus which had previously been the standard 2L treatment for advanced RCC since the 2008 RECORD 1 trial. It is notable that these studies were all conducted when the SOC for 1L advanced ccRCC were VEGF-TKIs. No clinical studies have so far been conducted to investigate subsequent treatment options following 1L treatment with IO/VEGF-TKI either in combination or in sequence. As a result, there is no standard of care (SOC) for patients with 2L+ post PD-(L)1 inhibitors/post VEGF-TKIs advanced RCC, and there remains an unmet need to improve treatment outcomes in this population.

### SUMMARY OF INVENTION

The invention is defined in the appended claims. Any references in the description to methods of treatment refer to the compounds, pharmaceutical compositions and medicaments of the present invention for use in a method for treatment of the human (or animal) body by therapy.

The present disclosure further provides kits including belzutifan and lenvatinib, or a pharmaceutically acceptable salt thereof.

Also provided herein are uses of a therapeutic combination for treating RCC, and the therapeutic combination includes belzutifan, and lenvatinib, or a pharmaceutically acceptable salt thereof.

In one aspect, provided herein is a method of treating RCC, comprising administering to a human patient in need thereof:
(a) belzutifan; and
(b) lenvatinib, or a pharmaceutically acceptable salt thereof.

In certain embodiments, the cancer is metastatic. In some embodiments, the cancer is relapsed. In other embodiments, the cancer is refractory. In yet other embodiments, the cancer is relapsed and refractory.

In one embodiment, the cancer is advanced RCC. In another embodiment, the cancer is metastatic RCC. In yet another embodiment, the cancer is relapsed RCC. In still another embodiment, the cancer is refractory RCC. In yet still another embodiment, the cancer is relapsed and refractory RCC. In yet still another embodiment, the cancer is VHL-deficient RCC.

In another aspect, provided herein is a kit comprising:
(a) belzutifan; and
(b) lenvatinib, or a pharmaceutically acceptable salt thereof.

In certain embodiments, the kit further comprises instructions for administering to a human patient belzutifan and lenvatinib, or a pharmaceutically acceptable salt thereof.

In still another aspect, provided herein is use of a therapeutic combination for treating RCC in a human patient, wherein the therapeutic combination comprises:
(a) belzutifan; and
(b) lenvatinib, or a pharmaceutically acceptable salt thereof.

In certain embodiments, the cancer is metastatic. In some embodiments, the cancer is relapsed. In other embodiments, the cancer is refractory. In yet other embodiments, the cancer is relapsed and refractory. In yet still another embodiment, the cancer is VHL-deficient RCC.

In one embodiment, the cancer is advanced RCC. In another embodiment, the RCC is advanced RCC with clear cell component (ccRCC). In yet another embodiment, the cancer is metastatic RCC. In yet another embodiment, the cancer is relapsed RCC. In still another embodiment, the cancer is refractory RCC. In yet still another embodiment, the cancer is relapsed and refractory RCC. In yet still another embodiment, the cancer is VHL-deficient RCC.

In one embodiment, the human patient has failed other treatments. In another embodiment, the human patient has advanced RCC with clear cell component (ccRCC) who has experienced disease progression on or after having received systemic treatment for advanced disease with PD-(L)1 checkpoint inhibitors and a VEGF-TKI (2L+ RCC).

In certain embodiments of various methods, kits, or uses provided herein, the HIF-2α inhibitor is belzutifan, or a pharmaceutically acceptable salt thereof.

In yet still another embodiment of various methods, kits, or uses provided herein, the lenvatinib or a pharmaceutically acceptable salt thereof is lenvatinib mesylate. Capsules for oral administration contain 4 mg or 10 mg of lenvatinib, equivalent to 4.90 mg or 12.25 mg of lenvatinib mesylate, respectively. In another embodiment, when a pharmaceutically acceptable salt of lenvatinib is administered, such as lenvatinib mesylate, and the dose of lenvatinib to be used is 4 mg, a medical practitioner would know to administer 4.90 mg of lenvatinib mesylate. In another embodiment, when a pharmaceutically acceptable salt of lenvatinib is administered, such as lenvatinib mesylate, and the dose of lenvatinib to be used is 10 mg, a medical practitioner would know to administer 12.25 mg of lenvatinib mesylate. In other embodiments of various methods described herein, the human patient is administered 8, 10, 12, 14, 18, 20, or 24 mg lenvatinib once daily.

In embodiments of various methods described herein, the HIF-2α inhibitor is belzutifan, or a pharmaceutically acceptable salt thereof, and the human patient is administered from about 40 mg to about 120 mg of belzutifan daily. In some embodiments, the human patient is administered 40, 80, or 120 mg of belzutifan once daily. In one specific embodiment, the human patient is administered 40 mg of belzutifan once daily. In another specific embodiment, the human patient is administered 80 mg of belzutifan once daily. In another specific embodiment, the human patient is administered 120 mg of belzutifan once daily.

Thus, in some embodiments, the human patient is administered:
(a) 40, 80, or 120 mg belzutifan once daily; and
(b) 8, 10, 12, 14, 18, 20, or 24 mg lenvatinib once daily.

In certain embodiments, the human patient is administered:
(a) 120 mg belzutifan once daily; and
(b) 20 mg lenvatinib once daily.

In a specific embodiment, provided herein is a method of treating RCC, comprising administering to a human patient in need thereof:
(a) 120 mg belzutifan once daily; and
(b) 20 mg lenvatinib once daily.

In certain embodiments of such a method, the HIF-2α inhibitor and lenvatinib are administered on the same day. In some embodiments, the HIF-2α inhibitor and lenvatinib are administered sequentially. In other embodiments, the HIF-2α inhibitor, and lenvatinib are administered concurrently.

In some embodiments of various methods, kits, or uses described herein, the pharmaceutically acceptable salt of lenvatinib-lenvatinib mesylate-can be used. When lenvatinib mesylate is used, the dosage of lenvatinib mesylate is appropriately adjusted to provide equal mole of lenvatinib as 8, 10, 12, 14, 18, 20, or 24 mg lenvatinib provides.

### BRIEF DESCRIPTION OF THE DRAWINGS

FIG. 1 illustrates a scheme of the design overview the Phase 1b/2 Study of belzutifan (MK-6482) in combination with lenvatinib (Experimental Arm B5) in patients with second-line plus (2L+) post programmed cell death 1/programmed cell death ligand 1 (PD-[L]1) inhibitor/post vascular endothelial growth factor-tyrosine kinase inhibitor (VEGF-TKI) advanced RCC with clear cell component (ccRCC).

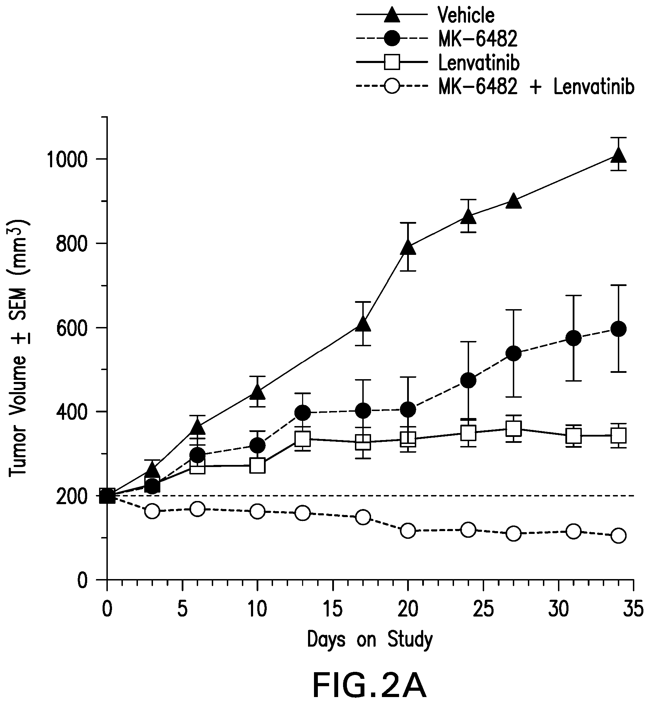

FIG. 2A illustrates the anti-tumor effect of concurrent administration of Lenvatinib and a HIF-2a inhibitor (MK-6482) as shown by average tumor volumes in each treatment group. Experimental details are described in Example 2.

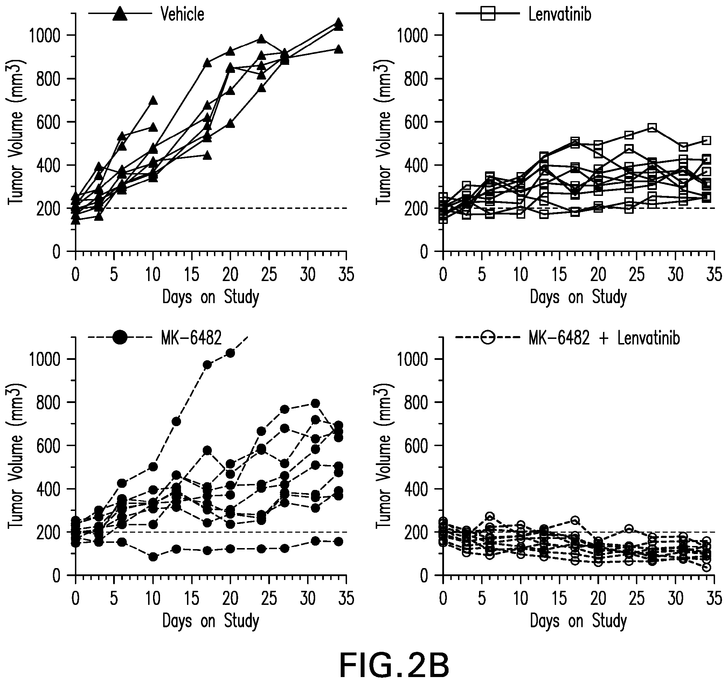

FIG. 2B illustrates the anti-tumor effect of concurrent administration of Lenvatinib and a HIF-2a inhibitor (MK-6482) as shown by average tumor volumes in the individual growth curves for each respective group. Experimental details are described in Example 2.

### DETAILED DESCRIPTION OF THE INVENTION

### 1. Definitions

Certain technical and scientific terms are specifically defined below. Unless specifically defined elsewhere in this document, all other technical and scientific terms used herein have the meaning commonly understood by one of ordinary skill in the art to which this disclosure relates.

"About" when used to modify a numerically defined parameter (*e.g*., the dose of an anti-PD-1 antibody or antigen binding fragment thereof, a HIF-2α inhibitor or antigen binding fragment thereof, or lenvatinib, or the length of treatment time with a combination therapy described herein) means that the parameter is within 20%, within15%, within 10%, within 9%, within 8%, within 7%, within 6%, within 5%, within 4%, within 3%, within 2%, within 1%, or less of the stated numerical value or range for that parameter; where appropriate, the stated parameter may be rounded to the nearest whole number. For example, a dose of about 5 mg/kg may vary between 4.5 mg/kg and 5.5 mg/kg.

As used herein, including the appended claims, the singular forms of words such as "a," "an," and "the," include their corresponding plural references unless the context clearly dictates otherwise.

The terms "administration" or "administer" refers to the act of injecting or otherwise physically delivering a substance as it exists outside the body *(e.g.,* a HIF-2α inhibitor and lenvatinib as described herein) into a patient, such as by oral, mucosal, intradermal, intravenous, subcutaneous, intramuscular delivery, and/or any other methods of physical delivery described herein or known in the art.

"HIF-2α inhibitor" means any chemical compound or biological molecule that inhibits the activity of HIF-2α. Alternative names or synonyms for HIF-2α include but are not limited to: hypoxia-inducible factor-2alpha, endothelial PAS domain-containing protein 1, and EPAS1.

As used herein, the terms "at least one" item or "one or more" item each include a single item selected from the list as well as mixtures of two or more items selected from the list.

As used herein, the term "immune response" relates to any one or more of the following: specific immune response, non-specific immune response, both specific and non-specific response, innate response, primary immune response, adaptive immunity, secondary immune response, memory immune response, immune cell activation, immune cell-proliferation, immune cell differentiation, and cytokine expression.

The term "subject" (alternatively "patient") as used herein refers to a mammal that has been the object of treatment, observation, or experiment. The mammal may be male or female. The mammal may be one or more selected from the group consisting of humans, bovine *(e.g.,* cows), porcine *(e.g.,* pigs), ovine *(e.g.,* sheep), capra *(e.g.,* goats), equine *(e.g.,* horses), canine *(e.g.,* domestic dogs), feline *(e.g.,* house cats), lagomorphs *(e.g.,* rabbits), rodents (*e.g.*, rats or mice), Procyon lotor (*e.g*., raccoons). In particular embodiments, the subject is human.

The term "subject in need thereof" as used herein refers to a subject diagnosed with or suspected of having cancer or an infectious disease as defined herein.

The therapeutic agents and compositions provided by the present disclosure can be administered via any suitable enteral route or parenteral route of administration. The term "enteral route" of administration refers to the administration via any part of the gastrointestinal tract. Examples of enteral routes include oral, mucosal, buccal, and rectal route, or intragastric route. "Parenteral route" of administration refers to a route of administration other than enteral route. Examples of parenteral routes of administration include intravenous, intramuscular, intradermal, intraperitoneal, intratumor, intravesical, intraarterial, intrathecal, intracapsular, intraorbital, intracardiac, transtracheal, intraarticular, subcapsular, subarachnoid, intraspinal, epidural and intrasternal, subcutaneous, or topical administration. The therapeutic agents and compositions of the disclosure can be administered using any suitable method, such as by oral ingestion, nasogastric tube, gastrostomy tube, injection, infusion, implantable infusion pump, and osmotic pump. The suitable route and method of administration may vary depending on a number of factors such as the specific therapeutic agent being used, the rate of absorption desired, specific formulation or dosage form used, type or severity of the disorder being treated, the specific site of action, and conditions of the patient, and can be readily selected by a person skilled in the art.

"RECIST 1.1 Response Criteria" as used herein means the definitions set forth in Eisenhauer, E.A. et al., Eur. J. Cancer 45:228-247 (2009) for target lesions or nontarget lesions, as appropriate based on the context in which response is being measured.

"Sustained response" means a sustained therapeutic effect after cessation of treatment as described herein. In some embodiments, the sustained response has a duration that is at least the same as the treatment duration, or at least 1.5, 2.0, 2.5 or 3 times longer than the treatment duration.

"Treat" or "treating" cancer as used herein means to administer a therapeutic combination of a HIF-2α inhibitor and lenvatinib or a pharmaceutically acceptable salt thereof, to a subject having cancer or diagnosed with cancer to achieve at least one positive therapeutic effect, such as, for example, reduced number of cancer cells, reduced tumor size, reduced rate of cancer cell infiltration into peripheral organs, or reduced rate of tumor metastasis or tumor growth. Such "treatment" may result in a slowing, interrupting, arresting, controlling, or stopping of the progression of cancer as described herein but does not necessarily indicate a total elimination of the cancer or the symptoms of the cancer. Positive therapeutic effects in cancer can be measured in a number of ways (*See*, W. A. Weber, J. Nucl. Med. 50:1S-10S (2009)). For example, with respect to tumor growth inhibition, according to NCI standards, a T/C ≦ 42% is the minimum level of anti-tumor activity. A T/C < 10% is considered a high anti-tumor activity level, with T/C (%) = Median tumor volume of the treated/Median tumor volume of the control × 100. In some embodiments, the treatment achieved by a combination therapy of the disclosure is any of PR, CR, OR, PFS, DFS, and OS. PFS, also referred to as "Time to Tumor Progression" indicates the length of time during and after treatment that the cancer does not grow, and includes the amount of time patients have experienced a CR or PR, as well as the amount of time patients have experienced SD. DFS refers to the length of time during and after treatment that the patient remains free of disease. OS refers to a prolongation in life expectancy as compared to naive or untreated individuals or patients. In some embodiments, response to a combination therapy of the disclosure is any of PR, CR, PFS, DFS, or OR that is assessed using RECIST 1.1 response criteria. The treatment regimen for a combination therapy of the disclosure that is effective to treat a cancer patient may vary according to factors such as the disease state, age, and weight of the patient, and the ability of the therapy to elicit an anti-cancer response in the subject. While an embodiment of any of the aspects of the disclosure may not be effective in achieving a positive therapeutic effect in every subject, it should do so in a statistically significant number of subjects as determined by any statistical test known in the art such as the Student's t-test, the chi²-test, the U-test according to Mann and Whitney, the Kruskal-Wallis test (H-test), Jonckheere-Terpstra-test and the Wilcoxon-test.

As used herein, the terms "combination," "combination therapy," and "therapeutic combination" refer to treatments in which at least one a HIF-2α inhibitor (being belzutifan) and lenvatinib or a pharmaceutically acceptable salt thereof, and optionally additional therapeutic agents, each are administered to a patient in a coordinated manner, over an overlapping period of time. The period of treatment with the HIF-2α inhibitor (the "HIF-2α inhibitor treatment") is the period of time that a patient undergoes treatment with the HIF-2α inhibitor; that is, the period of time from the initial dosing with the HIF-2α inhibitor through the final day of a treatment cycle. The period of treatment with lenvatinib or a pharmaceutically acceptable salt thereof (the "lenvatinib treatment") is the period of time that a patient undergoes treatment with lenvatinib; that is, the period of time from the initial dosing with lenvatinib through the final day of a treatment cycle. In the methods and therapeutic combinations described herein, the HIF-2α inhibitor treatment overlaps by at least one day with the lenvatinib treatment. In certain embodiments, the HIF-2α inhibitor treatment and the lenvatinib treatment are the same period of time. In some embodiments, the HIF-2α inhibitor treatment begins before the lenvatinib treatment. In other embodiments, the HIF-2α inhibitor treatment begins after the lenvatinib treatment. In certain embodiments, the HIF-2α inhibitor treatment is terminated prior to termination of the lenvatinib treatment. In certain embodiments, the lenvatinib treatment is terminated prior to termination of the HIF-2α inhibitor treatment.

The terms "treatment regimen," "dosing protocol," and "dosing regimen" are used interchangeably to refer to the dose and timing of administration of each therapeutic agent in a combination therapy of the disclosure.

"Tumor" as it applies to a subject diagnosed with, or suspected of having, a cancer refers to a malignant or potentially malignant neoplasm or tissue mass of any size, and includes primary tumors and secondary neoplasms. Non-limiting examples of tumors include solid tumor (*e.g*., sarcoma (such as chondrosarcoma), carcinoma (such as colon carcinoma), blastoma (such as hepatoblastoma), *etc.*) and blood tumor *(e.g.,* leukemia (such as acute myeloid leukemia (AML)), lymphoma (such as DLBCL), multiple myeloma (MM), *etc.*)*.*

The term "tumor volume" or "tumor size" refers to the total size of the tumor which can be measured as the length and width of a tumor. Tumor size may be determined by a variety of methods known in the art, such as, *e.g.,* by measuring the dimensions of tumor(s) upon removal from the subject, *e.g.,* using calipers, or while in the body using imaging techniques, *e.g.,* bone scan, ultrasound, CT or MRI scans.

Unless expressly stated to the contrary, all ranges cited herein are inclusive; *i.e.*, the range includes the values for the upper and lower limits of the range as well as all values in between. As an example, temperature ranges, percentages, ranges of equivalents, and the like described herein include the upper and lower limits of the range and any value in the continuum there between. Numerical values provided herein, and the use of the term "about", may include variations of ± 1%, ± 2%, ±3%, ± 4%, ± 5%, ± 10%, ± 15%, and ± 20% and their numerical equivalents. All ranges also are intended to include all included sub-ranges, although not necessarily explicitly set forth. For example, a range of 3 to 7 days is intended to include 3, 4, 5, 6, and 7 days. In addition, the term "or," as used herein, denotes alternatives that may, where appropriate, be combined; that is, the term "or" includes each listed alternative separately as well as their combination.

Where aspects or embodiments of the disclosure are described in terms of grouping of alternatives, the present disclosure encompasses not only the entire group listed as a whole, but each member of the group individually and all possible subgroups of the main group, but also the main group absent one or more of the group members. The present disclosure also envisages the explicit exclusion of one or more of any of the group members in the claims.

Unless otherwise defined, all technical and scientific terms used herein have the same meaning as commonly understood by one of ordinary skill in the art to which this disclosure relates. In case of conflict, the present specification, including definitions, will control. Throughout this specification and claims, the word "comprise," or variations such as "comprises" or "comprising" will be understood to imply the inclusion of a stated integer or group of integers but not the exclusion of any other integer or group of integers. Unless otherwise required by context, singular terms shall include pluralities and plural terms shall include the singular. Any example(s) following the term *"e.g."* or "for example" is not meant to be exhaustive or limiting.

Exemplary methods and materials are described herein, although methods and materials similar or equivalent to those described herein can also be used in the practice or testing of the present disclosure. The materials, methods, and examples are illustrative only and not intended to be limiting.

### 2. HIF-2α inhibitors

The HIF-2α inhibitor is belzutifan, or a pharmaceutically acceptable salt thereof, which is also known as MK-6482, PT2977, 3-[(1S,2S,3R)-2,3-difluoro-1-hydroxy-7-methylsulfonyl-indan-4-yl]oxy-5-fluoro-benzonitrile, and 3-[[(1S,2S,3R)-2,3-difluoro-2,3-dihydro-1-hydroxy-7-(methylsulfonyl)-1H-inden-4-yl]oxy]-5-fluorobenzonitrile and has the following chemical structure: 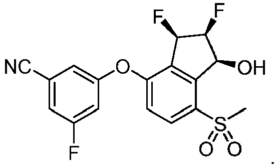 Belzutifan and its synthesis are described in U.S. Patent No. 9,969,689. Belzutifan as a potential treatment for clear cell renal cell carcinoma is described in Rui Xu et al., J. Med. Chem. 2019, 62, 6876-6891. Combinations of a PD-1/CTLA-4 inhibitor with a HIF-2α inhibitor for the treatment of melanoma, RCC or CRC are described in U.S. Patent No. 10,335,388. U.S. Publication 2018-0042884 describes the treatment of glioblastoma with a HIF-2α inhibitor. Oral formulations of belzutifan are described in International Application No. PCT/US2019/57725, which was filed on October 23, 2019.

### 3. Lenvatinib

Also provided herein is lenvatinib, which is a multiple RTK (multi-RTK) inhibitor that selectively inhibits the kinase activities of VEGF receptors.

Lenvatinib, which is also known as LENVIMA^{®}, Eisai Inc., Woodcliff Lake, NJ and 4-[3-chloro-4-(cyclopropylaminocarbonyl)aminophenoxy]-7-methoxy-6-quinolinecarboxamide, has the following chemical structure:

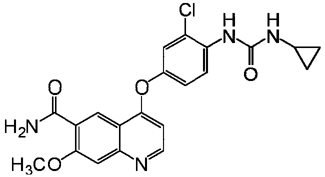

Levatinib, its synthesis and uses are described in U.S. Patent Nos. 7,253,286; 7,612,208; 9,006,256; 10,259,791; and 10,407,393.

### 4. Methods of Treating renal cell carcinoma (RCC) Using a Combination of belzutifan and lenvatinib or a pharmaceutically acceptable salt thereof

In certain embodiments, the method of treating RCC comprises administering to a human patient in need thereof:
(a) belzutifan; and
(b) lenvatinib, or a pharmaceutically acceptable salt thereof.

In certain embodiments, the cancer is metastatic. In some embodiments, the cancer is relapsed. In other embodiments, the cancer is refractory. In yet other embodiments, the cancer is relapsed and refractory.

In one embodiment, the cancer is advanced RCC. In another embodiment, the RCC is advanced RCC with clear cell component (ccRCC). In yet another embodiment, the cancer is metastatic RCC. In yet another embodiment, the cancer is relapsed RCC. In still another embodiment, the cancer is refractory RCC. In yet still another embodiment, the cancer is relapsed and refractory RCC. In yet still another embodiment, the cancer is VHL-deficient RCC.

In one embodiment, the human patient has failed other treatments. In another embodiment, the human patient has advanced RCC with clear cell component (ccRCC) who has experienced disease progression on or after having received systemic treatment for advanced disease with PD-(L)1 checkpoint inhibitors and a VEGF-TKI (2L+ RCC).

In some embodiments, provided herein is a method of treating advanced RCC, comprising administering to a human patient in need thereof:
(a) belzutifan; and
(b) lenvatinib, or a pharmaceutically acceptable salt thereof.

In some embodiments, provided herein is a method of treating advanced RCC with clear cell component, comprising administering to a human patient in need thereof:
(a) belzutifan; and
(b) lenvatinib,or a pharmaceutically acceptable salt thereof.

In other embodiments, provided herein is a method of treating metastatic RCC, comprising administering to a human patient in need thereof:
(a) belzutifan; and
(b) lenvatinib, or a pharmaceutically acceptable salt thereof.

In yet other embodiments, provided herein is a method of treating relapsed RCC, comprising administering to a human patient in need thereof:
(a) belzutifan; and
(b) lenvatinib, or a pharmaceutically acceptable salt thereof.

In yet still other embodiments, provided herein is a method of treating refractory RCC, comprising administering to a human patient in need thereof:
(a) belzutifan; and
(b) lenvatinib, or a pharmaceutically acceptable salt thereof.

In other embodiments, provided herein is a method of treating relapsed and refractory RCC, comprising administering to a human patient in need thereof:
(a) belzutifan; and
(b) lenvatinib, or a pharmaceutically acceptable salt thereof.

In other embodiments, provided herein is a method of treating VHL-deficient RCC, comprising administering to a human patient in need thereof:
(a) belzutifan; and
(b) lenvatinib, or a pharmaceutically acceptable salt thereof.

In one specific embodiment of various methods provided herein, the method for treating RCC comprises administering to a human patient in need thereof:
(a) belzutifan, or a pharmaceutically acceptable salt thereof; and
(b) lenvatinib, or a pharmaceutically acceptable salt thereof.

In one embodiment, the RCC is advanced RCC. In another embodiment, the RCC is advanced RCC with clear cell component. In yet another embodiment, the RCC is metastatic RCC. In yet another embodiment, the RCC is relapsed RCC. In still another embodiment, the RCC is refractory RCC. In yet still another embodiment, the RCC is relapsed and refractory RCC. In yet still another embodiment, the RCC is VHL-deficient RCC.

### 5. Dosing and Administration

Further provided herein are dosing regimens and routes of administration for treating RCC using a combination of belzutifan and lenvatinib or a pharmaceutically acceptable salt thereof.

Belzutifan or lenvatinib, or a pharmaceutically acceptable salt thereof, disclosed herein may be administered by doses administered, *e.g.*, daily, 1-7 times per week, weekly, bi-weekly, tri-weekly, every four weeks, every five weeks, every 6 weeks, monthly, bimonthly, quarterly, semiannually, annually, etc. Doses may be administered, e.g., intravenously, subcutaneously, topically, orally, nasally, rectally, intramuscular, intracerebrally, intraspinally, or by inhalation. In certain embodiments, the doses are administered intravenously. In certain embodiments, the doses are administered subcutaneously. In certain embodiments, the doses are administered orally. A total dose for a treatment interval is generally at least 0.05 µg/kg body weight, more generally at least 0.2 µg/kg, 0.5 µg/kg, 1 µg/kg, 10 µg/kg, 100 µg/kg, 0.25 mg/kg, 1.0 mg/kg, 2.0 mg/kg, 5.0 mg/ml, 10 mg/kg, 25 mg/kg, 50 mg/kg or more. Doses may also be provided to achieve a pre-determined target concentration of the antibody (*e.g*., anti-PD-1 antibody) or antigen binding fragment thereof in the subject's serum, such as 0.1, 0.3, 1, 3, 10, 30, 100, 300 µg/mL or more.

In embodiments of various methods described herein, the HIF-2α inhibitor is belzutifan or a pharmaceutically acceptable salt thereof and the human patient is administered from 40 to 120 mg once-daily. In still other embodiments of various methods described herein, 40, 80 or 120 mg of belzutifan is administered once-daily. In one specific embodiment, the human patient is administered 40 mg of belzutifan once-daily. In one specific embodiment, the human patient is administered 80 mg of belzutifan once-daily. In one specific embodiment, the human patient is administered 120 mg of belzutifan once-daily.

In certain embodiments, lenvatinib or a pharmaceutically acceptable salt thereof is administered orally. In some embodiments, lenvatinib or a pharmaceutically acceptable salt thereof is administered at a daily dose of 8, 10, 12, 14, 18, 20, or 24 mg, each as lenvatinib.

Thus, in some embodiments of various methods provided herein, the human patient is administered:
(a) 40, 80, or 120 mg belzutifan; and
(b) 8, 10, 12, 14, 18, 20, or 24 mg lenvatinib;
wherein (a) and (b) are administered daily.

In certain embodiments of various methods provided herein, the human patient is administered:
(a) 120 mg belzutifan; and
(b) 20 mg lenvatinib;
wherein (a) and (b) are administered daily.

In certain embodiments of various methods provided herein, the human patient is administered:
(a) 120 mg belzutifan; and
(b) 14 mg lenvatinib;
wherein (a) and (b) are administered daily.

In certain embodiments of various methods provided herein, the human patient is administered:
(a) 120 mg belzutifan; and
(b) 10 mg lenvatinib;
wherein (a) and (b) are administered daily.

In certain embodiments of various methods provided herein, the human patient is administered:
(a) 80 mg belzutifan; and
(b) 10 mg lenvatinib;
wherein (a) and (b) are administered daily.

In certain embodiments, the human patient has failed other treatments. In another embodiment, the human patient has advanced RCC with clear cell component (ccRCC) who has experienced disease progression on or after having received systemic treatment for advanced disease with PD-(L)1 checkpoint inhibitors and a VEGF-TKI (2L+ RCC).

In some embodiments, at least one of the therapeutic agents (*e.g.*, belzutifan or lenvatinib) in the combination therapy is administered using the same dosage regimen (dose, frequency, and duration of treatment) that is typically employed when the agent is used as monotherapy for treating the same condition. In other embodiments, the patient receives a lower total amount of at least one of the therapeutic agents (*e.g*., belzutifan or lenvatinib) in the combination therapy than when the agent is used as monotherapy, *e.g*., smaller doses, less frequent doses, and/or shorter treatment duration.

A combination therapy disclosed herein may be used prior to or following surgery to remove a tumor and may be used prior to, during, or after radiation treatment.

In some embodiments, a combination therapy disclosed herein is administered to a patient who has not previously been treated with a biotherapeutic or chemotherapeutic agent, *i.e.*, is treatment-naive. In other embodiments, the combination therapy is administered to a patient who failed to achieve a sustained response after prior therapy with the biotherapeutic or chemotherapeutic agent, *i.e.*, is treatment-experienced.

The therapeutic combination disclosed herein may be used in combination with one or more other active agents, including but not limited to, other anti-cancer agents that are used in the prevention, treatment, control, amelioration, or reduction of risk of a particular disease or condition (*e.g*., cancer). Such other active agents may be administered, by a route and in an amount commonly used therefor, contemporaneously or sequentially with one or more of the therapeutic agents in the combinations disclosed herein.

The one or more additional active agents may be co-administered with the belzutifan or lenvatinib or a pharmaceutically acceptable salt thereof. The additional active agent(s) can be administered in a single dosage form with one or more co-administered agent selected from belzutifan and lenvatinib or a pharmaceutically acceptable salt thereof. The additional active agent(s) can also be administered in separate dosage form(s) from the dosage forms containing belzutifan or lenvatinib or a pharmaceutically acceptable salt thereof.

### 6. Kits

In still another aspect, provided herein are kits comprising the therapeutic agents disclosed herein (*e.g*., belzutifan and lenvatinib) or pharmaceutical compositions thereof, packaged into suitable packaging material. A kit optionally includes a label or packaging insert that include a description of the components or instructions for use *in vitro*, *in vivo*, or *ex vivo*, of the components therein.

In some embodiments, the kit comprises
(a) belzutifan; and
(b) lenvatinib or a pharmaceutically acceptable salt thereof.

In certain embodiments, the kit further comprises instructions for administering to a human patient belzutifan and lenvatinib or a pharmaceutically acceptable salt thereof.

In one embodiment, the kit comprises: (a) one or more dosages of belzutifan, (b) one or more dosages of lenvatinib or a pharmaceutically acceptable salt thereof; and (c) instructions for administering to a human patient the belzutifan and lenvatinib or a pharmaceutically acceptable salt thereof.

The dosages for belzutifan or lenvatinib or a pharmaceutically acceptable salt thereof can be used in various kits herein. In some embodiments, a kit comprises dosages of each component sufficient for a certain period of treatment (*e.g*., 3, 6, 12, or 24 weeks, etc.). For example, a kit can comprise 21 dosages of 120 mg belzutifan, and 21 dosages of 20 mg lenvatinib (or equivalent amount of a pharmaceutically acceptable salt of lenvatinib), which are sufficient for a 3-week treatment. Or, a kit can also comprise 42 dosages of 120 mg belzutifan and 42 dosages of 20 mg lenvatinib (or equivalent amount of a pharmaceutically acceptable salt of lenvatinib), which are sufficient for a 6-week treatment.

In some embodiments, the kit comprises means for separately retaining the components, such as a container, divided bottle, or divided foil packet. A kit of this disclosure can be used for administration of different dosage forms, for example, oral and parenteral, for administration of the separate compositions at different dosage intervals, or for titration of the separate compositions against one another.

### 7. Uses of a Therapeutic Combination for Treating Renal cell cancer (RCC)

In still another aspect, provided herein are uses of a therapeutic combination for treating RCC in a human patient, wherein the therapeutic combination comprises:
(a) belzutifan; and
(b) lenvatinib,
or a pharmaceutically acceptable salt thereof.

In certain embodiments, the cancer is metastatic. In some embodiments, the cancer is relapsed. In other embodiments, the cancer is refractory. In yet other embodiments, the cancer is relapsed and refractory.

In one embodiment, the cancer is advanced RCC. In another embodiment, the cancer is advanced RCC with clear cell component. In yet another embodiment, the cancer is metastatic RCC. In yet another embodiment, the cancer is relapsed RCC. In still another embodiment, the cancer is refractory RCC. In yet still another embodiment, the cancer is relapsed and refractory RCC. In yet still another embodiment, the RCC is VHL-deficient RCC.

In one embodiment, provided herein is use of a therapeutic combination for treating RCC in a human patient, wherein the therapeutic combination comprises:
(a) belzutifan; and
(b) lenvatinib, or a pharmaceutically acceptable salt thereof.

In some embodiments, provided herein is use of a therapeutic combination for treating advanced RCC in a human patient, wherein the therapeutic combination comprises:
(a) belzutifan; and
(b) lenvatinib, or a pharmaceutically acceptable salt thereof.

In some embodiments, provided herein is use of a therapeutic combination for treating advanced RCC with clear cell component in a human patient, wherein the therapeutic combination comprises:
(a) belzutifan; and
(b) lenvatinib, or a pharmaceutically acceptable salt thereof.

In other embodiments, provided herein is use of a therapeutic combination for treating metastatic RCC in a human patient, wherein the therapeutic combination comprises:
(a) belzutifan; and
(b) lenvatinib, or a pharmaceutically acceptable salt thereof.

In yet other embodiments, provided herein is use of a therapeutic combination for treating relapsed RCC in a human patient, wherein the therapeutic combination comprises:
(a) belzutifan; and
(b) lenvatinib, or a pharmaceutically acceptable salt thereof.

In yet still other embodiments, provided herein is use of a therapeutic combination for treating refractory RCC in a human patient, wherein the therapeutic combination comprises:
(a) belzutifan; and
(b) lenvatinib, or a pharmaceutically acceptable salt thereof.

In other embodiments, provided herein is use of a therapeutic combination for treating relapsed and refractory RCC in a human patient, wherein the therapeutic combination comprises:
(a) belzutifan; and
(b) lenvatinib, or a pharmaceutically acceptable salt thereof.

In other embodiments, provided herein is use of a therapeutic combination for treating VHL-deficient RCC in a human patient, wherein the therapeutic combination comprises:
(a) belzutifan; and
(b) lenvatinib, or a pharmaceutically acceptable salt thereof.

In one specific embodiment, provided herein is use of a therapeutic combination for treating RCC, wherein the therapeutic combination comprises:
(a) belzutifan, or a pharmaceutically acceptable salt thereof; and
(b) lenvatinib, or a pharmaceutically acceptable salt thereof.

In one embodiment, the human patient has failed other treatments. In another embodiment, the human patient has advanced RCC with clear cell component (ccRCC) who has experienced disease progression on or after having received systemic treatment for advanced disease with PD-(L)1 checkpoint inhibitors and a VEGF-TKI (2L+ RCC). advanced RCC with clear cell component (ccRCC) who has experienced disease progression on or after having received systemic treatment for advanced disease with PD-(L)1 checkpoint inhibitors and a VEGF-TKI (2L+ RCC).

In one embodiment, the RCC is advanced RCC. In another embodiment, the RCC is advanced RCC with clear cell component. In yet another embodiment, the RCC is metastatic RCC. In yet another embodiment, the RCC is relapsed RCC. In still another embodiment, the RCC is refractory RCC. In yet still another embodiment, the RCC is relapsed and refractory RCC. In yet still another embodiment, the RCC is VHL-deficient RCC.

A number of embodiments of the invention have been described. It will be further understood that each embodiment may be combined with one or more other embodiments, to the extent that such a combination is consistent with the description of the embodiments.

### EXAMPLES

The examples in this section (section VI) are offered by way of illustration, and not by way of limitation.

### EXAMPLE 1

### Clinical Trial of Administering Belzutifan (MK-6482) and Lenvatinib to patients with advanced RCC with clear cell component (ccRCC) who have experienced disease progression on or after having received systemic treatment for advanced disease with PD-(L)1 checkpoint inhibitors and a VEGF-TKI (2L+ RCC).

As of September 6, 2019, a total of 34 healthy volunteers and 185 patients had been treated with MK-6482 in five ongoing clinical studies.

In an an ongoing randomized, single-dose, 2 period, 2 sequence cross-over Phase 1 study in 16 healthy female adult volunteers, the effect of food on the PK of a single dose of 120 mg MK-6482 was investigated. This study indicated that a high fat, high calorie meal did not affect the extent of MK-6482 exposure but lowered the maximum plasma MK-6482 concentration by approximately 35% and delayed time to peak MK-6482 exposure with a median difference (Fed - Fasted) of 2 hours. These data are not considered to be clinically meaningful and support dosing of MK-6482 with or without food. The most common adverse event (AE) reported was headache (12.5%).

A FIH Phase 1 study designed to assess the tolerability, safety, PK, and PD properties of MK-6482 in participants with various advanced solid tumors is ongoing. As of 06-SEP-2019 a total of 104 participants had been enrolled, including 43 participants with various advanced solid tumors in the dose-escalation portion (Part 1A) ranging from 20 to 240 mg QD and 120 mg BID. The MTD was not reached and 2 treatment-related DLTs were observed: 1 Grade 4 event of thrombocytopenia in the 240 mg QD cohort, and 1 Grade 3 event of hypoxia in the 120 mg BID cohort. The 120 mg QD dose was selected for further clinical development based on favorable PK, pharmacodynamic, and safety findings. 52 additional participants with advanced RCC were treated in an expansion cohort (Part 1B) at the clinical dose of 120 mg QD. In the combined dose escalation and expansion cohorts, the most common AEs (occurring in ≥20% of participants) were anemia, fatigue, dyspnea, nausea, and peripheral edema. The most common Grade 3 AEs were anemia and hypoxia (in ≥5% of participants). The median tₘₐₓ for MK-6482 was 1 to 2.8 hours and exposure increased with dose. The mean steady state t_{1/2} in the 120 mg QD expansion cohort (Part 1B) on Day 15 was 15.4 hours, resulting in a 1.5-fold accumulation from Day 1 to Day 15. The mean steady state Cₘₐₓ in the 120 mg QD expansion cohort (Part 1B) on Day 15 was 1.79 µg/mL (4.67 µM). The estimated CL/F was 5.22 to 14.4 L/hr. The estimated Vz/F was 106 to 266 L, which suggests extensive distribution to peripheral tissues. The CV was 32 to 59% for Cₘₐₓ and 24 to 48% for AUC after a single dose, and 27 to 56% for Cmax and 30 to 64% for AUC at steady state. In total, 55 participants with previously treated advanced RCC have been treated in this study with MK-6482 at 120 mg QD (3 patients in the dose-escalation portion of the study and 52 participants in the dose expansion portion of the study). Best response among these 55 participants included 11 participants (20%) with PR and 32 participants (58%) with SD as assessed by RECIST v1.1.

A Phase 2, open-label efficacy and safety study in participants with VHL disease associated RCC is ongoing. As of 06-SEP-2019, 61 participants had been enrolled at a dose of 120 mg QD. Efficacy data are not yet available. Fatigue was the most common AE of ≥ Grade 3 toxicity (reported by ≥5% of participants).

In addition, 20 patients with ccRCC are being evaluated in a Phase 2 Study, and 18 healthy adult volunteers are being evaluated in a Phase 1 bioavailability study.

Based on the data from these studies, the combination of 120 mg MK-6482 and 20 mg of lenvatinib will be evaluated in patients (2L) with advanced RCC with clear cell component (ccRCC) who have experienced disease progression on or after having received systemic treatment for advanced disease with PD-(L)1 checkpoint inhibitors and a VEGF-TKI (2L+ RCC).

The primary efficacy objective of this substudy is to evaluate the antitumor effect of belzutifan and lenvatinib in participants with advanced ccRCC. Specifically, the study will include male and female participants who are at least 18 years of age with 2L+ post PD-(L)1 inhibitors/post VEGF-TKIs advanced RCC with clear cell component (ccRCC). This substudy will use ORR as the primary efficacy endpoint. ORR is defined as the percentage of participants who achieve a confirmed CR or PR per RECIST 1.1 as assessed by BICR. Responses are based on BICR using RECIST 1.1, modified to follow a maximum of 10 target lesions and a maximum of 5 target lesions per organ. ORR is an appropriate endpoint to evaluate the antitumor activity of reference and experimental arms. Treatment effect measured by ORR can represent direct clinical benefit based on the specific disease, context of use, magnitude of the effect, number of CRs, durability of response, disease setting, location of the tumors, available therapy, and risk-benefit relationship.

This substudy will use DOR, PFS, OS, and CBR as secondary efficacy endpoints. DOR is defined as the time from the first documented evidence of CR or PR until disease progression or death due to any cause, whichever occurs first. DOR per RECIST 1.1, modified to follow a maximum of 10 target lesions and a maximum of 5 target lesions per organ, assessed by BICR will serve as an additional measure of efficacy and is a commonly accepted endpoint by both regulatory authorities and the oncology community.

PFS is defined as the time from the date of randomization to the first documented PD per RECIST 1.1 by BICR, or death due to any cause, whichever occurs first. Images will be read by a BICR to minimize bias in the response assessments. A PFS event can reflect tumor growth and be assessed before the determination of a survival benefit. Its determination is not confounded by subsequent therapy. Treatment effect measured by PFS can be a surrogate endpoint to represent direct clinical benefit based on the specific disease, context of use, magnitude of the effect, the disease setting, location of metastatic sites, available therapy, the risk-benefit relationship, and the clinical consequences of delaying or preventing progression in key disease sites (eg, delay of new lesions in the brain or spine) or delaying administration of more toxic therapies.

OS has been recognized as the gold standard for the demonstration of superiority of a new antineoplastic therapy in randomized clinical studies. OS is defined as the time from the date of randomization to the date of death from any cause.

CBR is a secondary endpoint commonly used in many cancer clinical trials {059M4P} and is defined as the percentage of participants who have achieved SD of ≥6 months or CR or PR based on assessments by BICR per RECIST 1.1.

Tumor size change will be an exploratory efficacy endpoint and is a proposed intermediate endpoint that may detect signals of early antitumor activity {059MRJ} and is defined as the sum of target lesions in longest diameter at each post-baseline assessment and change (and % change) from baseline.
Male/Female participants with histologically confirmed diagnosis of advanced clear cell RCC (with or without sarcomatoid features), who are at least 18 years of age will be enrolled in this study. A participant will be eligible for inclusion in the study if the participant:
1. Must have a histologically confirmed diagnosis of locally advanced/metastatic ccRCC (with or without sarcomatoid features), ie, Stage IV RCC per AJCC.
2. Have experienced disease progression on or after having received systemic treatment for locally advanced or metastatic RCC with both: PD-(L)1 checkpoint inhibitor and VEGF-TKI in sequence or in combination.
   PD-(L)1 checkpoint inhibitor treatment progression is defined by meeting ALL of the following criteria:
   - Has received at least 2 doses of an anti-PD-(L)1 mAb.
   - Has demonstrated PD during or after an anti-PD-(L)1 mAb as defined by RECIST 1.1. by investigator.
   - PD has been documented within 12 weeks from the last dose of an anti-PD-(L)1 mAb.
   VEGF-TKI treatment progression is defined by meeting the following criterion:
   - Has demonstrated PD during or after a treatment with a VEGF-TKI as defined by RECIST 1.1. by investigator.
3. Have measurable disease per RECIST 1.1 as assessed by BICR. Lesions situated in a previously irradiated area are considered measurable if progression has been demonstrated in such lesions.

The reference arm of the study is described below:

| **Intervention Name** | **Dose Formulation** | **Unit Dose Strength(s)** | **Dosage Level(s)** | **Route of Administration** | **Regimen/ Treatment Period** |
|---|---|---|---|---|---|
| Pembrolizumab | Solution for Infusion | 25 mg/mL | 400 mg | IV Infusion | Q6W |
| Lenvatinib | Capsule | 10 mg and 4 mg | 20 mg | Oral | QD |

| | | | | | |
|---|---|---|---|---|---|
| IV=intravenous; Q6W=every 6 weeks; QD=once a day NOTE: PEMBROLIZUMAB IN THIS ARM WILL BE ADMINISTERED AS AN IV INFUSION OVER 30 MINUTES. LENVATINIB WILL BE ADMINISTERED 30 MINUTES AFTER INFUSION IS COMPLETE | | | | | |

THE INVESTIGATIONAL AGENTS (ARM B5 ) FOR THE STUDY ARE DESCRIBED BELOW:

| **Arm Name** | **Intervention Name** | **Dose Formulation** | **Unit Dose Strengths** | **Dosage Levels** | **Route of Administration** | **Regimen/ Treatment Period** |
|---|---|---|---|---|---|---|
| Arm B5 | MK-6482 ^{d} | Tablet | 40 mg | 120 mg | Oral | QD |
| | Lenvatinib ^{f} | Capsule | 10 mg and 4 mg | 20 mg | Oral | QD |

| | | | | | | |
|---|---|---|---|---|---|---|
| ^{d} MK-6482 will be administered 30 minutes after any infusion (if applicable) is complete. ^{f} Lenvatinib will be administered last, 30 minutes after any other agents have been administered (ie: 30 minutes after infusion(s) are complete and/or oral medication has been administered. QD=once a day | | | | | | |

### MEDICATION DOSE LEVELS FOR EXPERIMENTAL ARM B5:

| **Arm B5** | **Dose Level 0** | **Dose Level -1** | **Dose Level -2** | **Dose Level -3** |
|---|---|---|---|---|
| Lenvatinib | 20 mg | 14 mg | 10 mg | 10 mg |
| MK-6482 | 120 mg | 120 mg | 120 mg | 80 mg |

### EXAMPLE 2

### VHL-deficient renal cell carcinoma xenograft tumor model combining a HIF-2α inhibitor (MK-6482) and VEGF tyrosine kinase inhibitor (lenvatinib).

Here we provide preclinical data using a human VHL-deficient renal cell carcinoma xenograft tumor model to demonstrate the anti-tumor benefit from combining a HIF-2α inhibitor (MK-6482) and VEGF tyrosine kinase inhibitor (lenvatinib).

Prior to treatment initiation, female SCID Beige mice aged 7 weeks weighing between 18 to 21 grams were anesthetized and inoculated subcutaneously on the rear flank with single cell suspension of approximately 95% viable log-phase sub-confluent UMRC2 (5.0 x 10⁶) cells in 0.1 mL of serum-free Dulbecco's Modified Eagle Medium (DMEM) to initiate tumor development. Treatment was initiated when the mean tumor volume of inoculated animals reached approximately 205mm³. Mice were pair-matched into 4 treatment groups consisting of 10 mice per group. Treatment groups consisted of: 1) 0.5% methylcellulose + 0.5% Tween 80 (Vehicle); 2) MK-6482; 3) Lenvatinib; 4) MK-6482 + Lenvatinib. Vehicle and MK-6482 were orally gavage-dosed twice daily (BID) at 3 mg/kg body weight. Lenvatinib was orally dosed once daily (QD) at 10 mg/kg body weight. Treatment began on Day 0 and completed on Day 34. -Caliper measurements of tumors and body weights were captured twice weekly. Statistical analysis was performed by one-way ANOVA with Tukey's multiple comparisons tests at the end of the study, Day 34.

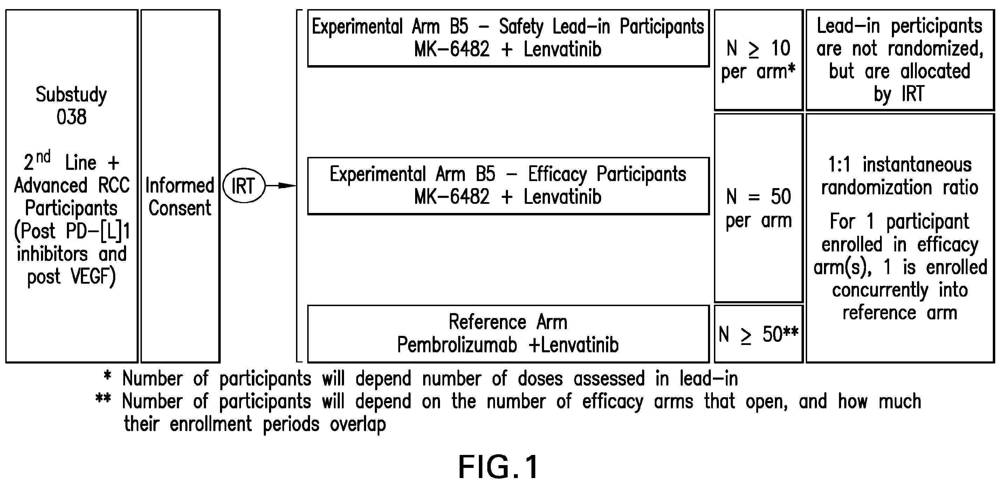

As shown in Figures 1A & 1B, the mean anti-tumor response of combination therapy with MK-6482 + lenvatinib was greater than MK-6482 monotherapy (p<0.0001) or lenvatinib monotherapy (p=0.017). All treatment groups demonstrated significant anti-tumor activity compared to the vehicle control group including MK-6482 monotherapy (p=0.003), lenvatinib monotherapy (p<0.0001), and MK-6482 + lenvatinib (p<0.0001). A summary of tumor growth inhibition (TGI) of treatment groups respective to Vehicle-treated animals at Day 34 and observations of partial tumor regressions (PRs) or complete tumor regressions (CRs) at the end of the study is provided in the table below:

| **Treatment** | **TGI (p-value)** | **Final PRs or CRs** |
|---|---|---|
| Lenvatinib | 83% (p<0.0001) | 0/10 PRs, 0/10 CRs |
| MK-6482 | 51% (p=0.058) | 1/10 PRs, 0/10 CRs |
| MK-6482 + Lenvatinib | 112% (p<0.0001) | 10/10 PRs, 0/10 CRs, |

CRs were defined as no observable tumor whereas PRs were tumors whose volume was lower than the original tumor size when treatment began. No new safety signals were observed in preclinical studies for the combination of lenvatinib + MK-6482 as assessed by changes in body weight or early mortality.

As shown in the results provided above, treatment with the combination of lenvatinib + MK-6482 is advantageous over treatment with each agent when administered alone.

## Claims

1. A combination of:
(a) belzutifan, or a pharmaceutically acceptable salt thereof; and
(b) lenvatinib, or a pharmaceutically acceptable salt thereof for use in a method of treating cancer, wherein the cancer is renal cell carcinoma (RCC), the method comprising administering the combination to a human patient in need thereof.

2. The combination for use according to claim 1, wherein the RCC is advanced RCC.

3. The combination for use according to claim 2, wherein the RCC is advanced RCC with clear cell component (ccRCC).

4. The combination for use according to claim 3, wherein the human patient has advanced RCC with clear cell component (ccRCC) who has experienced disease progression on or after having received systemic treatment for advanced disease with PD-(L)1 checkpoint inhibitors and a VEGF-TKI (2L+ RCC).

5. The combination for use according to any one of claims 2-4, wherein the RCC is metastatic RCC.

6. The combination for use according to any one of claims 2-4, wherein the RCC is VHL-deficient RCC.

7. A kit comprising:
(a) belzutifan, or a pharmaceutically acceptable salt thereof; and
(b) lenvatinib, or a pharmaceutically acceptable salt thereof.

8. The kit of claim 7, further comprising instructions for administering to a human patient the belzutifan, or a pharmaceutically acceptable salt thereof and lenvatinib, or a pharmaceutically acceptable salt thereof.

9. The combination for use according to any one of claims 1-6, wherein the human patient is administered from 40 mg to 120 mg of belzutifan, and wherein belzutifan is administered once-daily.

10. The combination for use according to claim 9, wherein the human patient is administered 40, 80, or 120 mg of belzutifan, and wherein belzutifan is administered once-daily.

11. The combination for use according to claim 10, wherein the human patient is administered 120 mg of belzutifan.

12. The combination for use according to any one of claims 1-6 or 9-11, wherein the human patient is administered 8, 10, 12, 14, 18, 20, or 24 mg lenvatinib, and wherein lenvatinib is administered once daily.

13. A combination of:
(a) 120 mg of belzutifan; and
(b) 20 mg lenvatinib
for use in a method of treating RCC, the method comprising administering the combination to a human patient in need thereof.

14. The combination for use according to claim 13, wherein (a) and (b) are administered once daily.

15. The combination for use according to any one of claims 13-14, wherein (a) and (b) are administered on the same day, and wherein (a) and (b) are administered sequentially or concurrently.

16. The combination for use or the kit according to any one of claims 1-15, wherein (b) is lenvatinib mesylate.

## Patentansprüche

1. Kombination von:
(a) Belzutifan oder einem pharmazeutisch unbedenklichen Salz davon; und
(b) Lenvatinib oder einem pharmazeutisch unbedenklichen Salz davon zur Verwendung bei einem Verfahren zur Behandlung von Krebs, wobei es sich bei dem Krebs um Nierenzellkarzinom (Renal Cell Carcinoma, RCC) handelt, wobei das Verfahren Verabreichen der Kombination an einen diese benötigenden menschlichen Patienten umfasst.

2. Kombination zur Verwendung nach Anspruch 1, wobei es sich bei dem RCC um fortgeschrittenes RCC handelt.

3. Kombination zur Verwendung nach Anspruch 2, wobei es sich bei dem RCC um fortgeschrittenes RCC mit Klarzellkomponente (ccRCC) handelt.

4. Kombination zur Verwendung nach Anspruch 3, wobei bei dem menschlichen Patienten fortgeschrittenes RCC mit Klarzellkomponente (ccRCC) vorliegt und die Krankheit bei oder nach Erhalten einer systemischen Behandlung gegen fortgeschrittene Krankheit mit PD-(L)1-Checkpoint-Inhibitoren und einem VEGF-TKI fortgeschritten ist (2L+ RCC).

5. Kombination zur Verwendung nach einem der Ansprüche 2-4, wobei es sich bei dem RCC um metastasiertes RCC handelt.

6. Kombination zur Verwendung nach einem der Ansprüche 2-4, wobei es sich bei dem RCC um RCC mit VHL-Defizienz handelt.

7. Kit, umfassend:
(a) Belzutifan oder ein pharmazeutisch unbedenkliches Salz davon; und
(b) Lenvatinib oder ein pharmazeutisch unbedenkliches Salz davon.

8. Kit nach Anspruch 7, ferner umfassend Anweisungen zur Verabreichung des Belzutifan oder eines pharmazeutisch unbedenklichen Salzes davon und Lenvatinib oder eines pharmazeutisch unbedenklichen Salzes davon an einen menschlichen Patienten.

9. Kombination zur Verwendung nach einem der Ansprüche 1-6, wobei dem menschlichen Patienten 40 mg bis 120 mg Belzutifan verabreicht werden und wobei Belzutifan einmal täglich verabreicht wird.

10. Kombination zur Verwendung nach Anspruch 9, wobei dem menschlichen Patienten 40, 80 oder 120 mg Belzutifan verabreicht werden und wobei Belzutifan einmal täglich verabreicht wird.

11. Kombination zur Verwendung nach Anspruch 10, wobei dem menschlichen Patienten 120 mg Belzutifan verabreicht werden.

12. Kombination zur Verwendung nach einem der Ansprüche 1-6 oder 9-11, wobei dem menschlichen Patienten 8, 10, 12, 14, 18, 20 oder 24 mg Lenvatinib verabreicht werden und wobei Lenvatinib einmal täglich verabreicht wird.

13. Kombination von:
(a) 120 mg Belzutifan; und
(b) 20 mg Lenvatinib
zur Verwendung bei einem Verfahren zur Behandlung von RCC, wobei das Verfahren Verabreichen der Kombination an einen diese benötigenden menschlichen Patienten umfasst.

14. Kombination zur Verwendung nach Anspruch 13, wobei (a) und (b) einmal täglich verabreicht werden.

15. Kombination zur Verwendung nach einem der Ansprüche 13-14, wobei (a) und (b) am gleichen Tag verabreicht werden und wobei (a) und (b) nacheinander oder gleichzeitig verabreicht werden.

16. Kombination zur Verwendung oder Kit nach einem der Ansprüche 1-15, wobei es sich bei (b) um Lenvatinib-Mesylat handelt.

## Revendications

1. Association de :
(a) belzutifan, ou un sel pharmaceutiquement acceptable de celui-ci ; et
(b) lenvatinib, ou un sel pharmaceutiquement acceptable de celui-ci, destinée à être utilisée dans une méthode de traitement de cancer, le cancer étant un carcinome à cellules rénales (CCR), la méthode comprenant l'administration de l'association à un patient humain qui en a besoin.

2. Association destinée à être utilisée selon la revendication 1, le CCR étant un CCR avancé.

3. Association destinée à être utilisée selon la revendication 2, le CCR étant un CCR avancé avec composante à cellules claires (CCRcc).

4. Association destinée à être utilisée selon la revendication 3, le patient humain ayant un CCR avancé avec composante à cellules claires (CCRcc) ayant connu une progression de la maladie pendant ou après qu'il a reçu un traitement systémique pour la maladie avancée avec des inhibiteurs de point de contrôle PD-(L)1 et un VEGF-TKI (CCR 2L+).

5. Association destinée à être utilisée selon l'une quelconque des revendications 2 à 4, le CCR étant un CCR métastatique.

6. Association destinée à être utilisée selon l'une quelconque des revendications 2 à 4, le CCR étant un CCR déficient en VHL.

7. Kit comprenant :
(a) du belzutifan, ou un sel pharmaceutiquement acceptable de celui-ci ; et
(b) du lenvatinib ou un sel pharmaceutiquement acceptable de celui-ci.

8. Kit selon la revendication 7, comprenant en outre des instructions pour l'administration à un patient humain du belzutifan, ou d'un sel pharmaceutiquement acceptable de celui-ci, et de lenvatinib, ou d'un sel pharmaceutiquement acceptable de celui-ci.

9. Association destinée à être utilisée selon l'une quelconque des revendications 1 à 6, de 40 mg à 120 mg de belzutifan étant administré au patient humain et du belzutifan étant administré une fois par jour.

10. Association destinée à être utilisée selon la revendication 9, 40, 80 ou 120 mg de belzutifan étant administré au patient humain et du belzutifan étant administré une fois par jour.

11. Association destinée à être utilisée selon la revendication 10, 120 mg de belzutifan étant administré au patient humain.

12. Association destinée à être utilisée selon l'une quelconque des revendications 1 à 6 et 9 à 11, 8, 10, 12, 14, 18, 20 ou 24 mg de lenvatinib étant administré au patient humain et du lenvatinib étant administré une fois par jour.

13. Association de :
(a) 120 mg de belzutifan ; et
(b) 20 mg de lenvatinib
destinée à être utilisée dans une méthode de traitement de CCR, la méthode comprenant l'administration de l'association à un patient humain qui en a besoin.

14. Association destinée à être utilisée selon la revendication 13, (a) et (b) étant administrés une fois par jour.

15. Association destinée à être utilisée selon l'une quelconque des revendications 13 à 14, (a) et (b) étant administrés le même jour et (a) et (b) étant administrés séquentiellement ou simultanément.

16. Association destinée à être utilisée ou kit selon l'une quelconque des revendications 1 à 15, (b) étant le mésylate de lenvatinib.
